(19) Europäisches Patentamt
European Patent Office
Office européen des brevets

(11) **EP 4 289 364 A1**

(12) **EUROPEAN PATENT APPLICATION**
published in accordance with Art. 153(4) EPC

(43) Date of publication:
**13.12.2023 Bulletin 2023/50**

(21) Application number: **21924098.3**

(22) Date of filing: **30.06.2021**

(51) International Patent Classification (IPC):
**A61B 8/00** (2006.01)   **A61B 8/06** (2006.01)

(86) International application number:
**PCT/CN2021/103789**

(87) International publication number:
**WO 2022/166091 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.02.2021   CN 202110184305**

(71) Applicant: **Eieling Technology Limited
Hong Kong (HK)**

(72) Inventors:
• **HUANG, Zihao
  Hong Kong Science Park, NT, Hong Kong (CN)**
• **WANG, Like
  Hong Kong Science Park, NT, Hong Kong (CN)**
• **ZHENG, Yongping
  Hong Kong Science Park, NT, Hong Kong (CN)**
• **CHENG, Lok Kan
  Hong Kong Science Park, NT, Hong Kong (CN)**

(74) Representative: **Puschmann Borchert Kaiser
Klettner
Patentanwälte Partnerschaft mbB
Bajuwarenring 21
82041 Oberhaching (DE)**

(54) **METHOD AND APPARATUS FOR EVALUATING CONTACT STATE OF ULTRASOUND PROBE ON BASIS OF SOFT TISSUE MORPHOLOGY**

(57)    A method for evaluating a contact state of an ultrasound probe (308, 802, 901) on the basis of soft tissue morphology. The method comprises the steps of: S11: collecting ultrasound information of morphological responses of an object under test (307) for different contact states; S12: on the basis of a feature of soft tissue and a boundary feature of the soft tissue in the ultrasound information, recognizing a boundary position of the soft tissue in the ultrasound information; S13: on the basis of the boundary position and a preset area division condition, determining an area of interest (301) and a sampling sub-area in the area of interest (301); S14: extracting a morphological parameter of the soft tissue from the selected area of interest (301); S15: according to the morphological parameter, calculating a predicted value that represents the contact state of an ultrasound probe (308, 802, 901) by using at least one morphological response of the soft tissue; and S16: converting the predicted value into a time-varying indication signal. On the basis of an existing ultrasound imaging apparatus, and by using software technical means, an apparatus for evaluating a contact state of an ultrasound probe (308, 802, 901) on the basis of soft tissue morphology can accurately calculate the contact state of the ultrasound probe (308, 802, 901) without needing to change the hardware, thereby facilitating the operation of the apparatus by an operator.

| |
|---|
| collecting ultrasound information of morphological responses of an object under test for different contact states — S11 |
| on the basis of a feature of soft tissue and a boundary feature of the soft tissue in the ultrasound information, recognizing a boundary position of the soft tissue in the ultrasound information — S12 |
| on the basis of the boundary position and a preset area division condition, determining an area of interest and a sampling sub-area in the area of interest — S13 |
| extracting a morphological parameter of the soft tissue from the selected area of interest — S14 |
| according to the morphological parameter, calculating a predicted value that represents the contact state of an ultrasound probe by using at least one morphological response of the soft tissue — S15 |
| converting the predicted value into a time-varying indication signal — S16 |

Figure 1

EP 4 289 364 A1

## Description

## TECHNICAL FIELD

The present application relates to the technical field of ultrasonic testing, in particular to a method and apparatus for evaluating the contact state of ultrasound probe based on the basis of soft tissue morphology

## BACKGROUND

The contact state of a medical ultrasound probe is an important influencing factor in quality control, whether it is for diagnostic ultrasound imaging or therapeutic ultrasound physical therapy. The contact state typically includes the pressure applied by the ultrasound probe on the surface of the human body and the perpendicularity of the probe. For therapeutic ultrasound, the effectiveness of the treatment depends on the ultrasound intensity that ultimately enters the body, which is related to the alignment between the probe and the skin. For diagnostic ultrasound, the contact state of the probe significantly affects the imaging quality and the reliability of functional measurements. In particular, for quantitative ultrasound, standardized procedures (i.e., maintaining consistent pressure and tilt angle between the probe and the skin) are essential for repeated measurements. Quantitative ultrasound is the use of ultrasound waves to quantitatively assess the structure and physiological characteristics of biological tissues. It is gradually being applied in clinical and research fields. Specific examples include measuring liver stiffness and assessing the degree of liver fibrosis and steatosis using ultrasound; extracting muscle structure data to evaluate the contraction function of target muscles; measuring vascular morphology and hemodynamic characteristics to assess vascular function. However, research and extensive practice have shown that probe pressure can alter the skin-to-liver capsule distance (SCD), leading to a decrease in the success rate of liver elasticity detection. Different probe forces and tilt angles can cause passive deformation of soft tissues in ultrasound images, resulting in an underestimation of the true muscle geometry. The probe may also compress superficial peripheral vessels, leading to changes in vessel diameter and wall thickness (especially veins), which can affect measurement results and the diagnosis of vascular lesions.

Indeed, the traditional approach to assessing contact state has been largely based on empirical judgment. Operators have had to subjectively assess the contact state between the probe and the body surface, leading to low reliability in their actions. This is evident in the significant variability in results obtained by different operators or even the same operator under different conditions. Taking liver elasticity detection as an example, obtaining accurate elasticity readings requires operators to apply appropriate pressure in the intercostal spaces. However, purely manual operations cannot guarantee that the probe pressure remains within the desired range. If the pressure is too low, ultrasound waves and shear waves generated through external vibrations are prone to attenuation, making it difficult for them to propagate effectively within the liver. If the pressure is too large, excessive pressure can cause discomfort to the subject.

Some engineering methods have been employed to quantify the contact state of probes. In existing designs, an instantaneous elasticity detection device has been proposed, which places a force sensor between the ultrasound transducer and the vibrator to measure the pressure applied to the contact surface of the probe. Another approach involves using position sensors to measure the displacement of an internal spring, indirectly measuring the applied force. However, these methods can only estimate the probe pressure based on external information obtained from additional devices and cannot reflect its true impact on the object under test. Indeed, there are variations in body size and anatomy among individuals. Muscle thickness, fat content, and skin tightness differ between individuals who are thin or obese, and there are also variations in different anatomical regions. It is generally believed that accurate detection results can only be obtained when the applied pressure on a specific organ falls within the corresponding pressure range for that organ. Therefore, universal measurement ranges and standards are not universally applicable to measuring different populations and anatomical sites.

Therefore, in the field of ultrasonic detection technology, there is a lack of a mechanism that can specifically indicate the contact state between the ultrasound probe and the surface of the tested tissue based on individual differences in tissue structure.

## SUMMARY

The purpose of this application is to address the shortcomings of the aforementioned technical solution and provide a method for evaluating a contact state of an ultrasound probe on the basis of soft tissue morphology. The method includes collecting ultrasound information of morphological responses of an object under test for different contact states; determining boundary positions of at least one type of soft tissue in the object under test based on the feature of at least one soft tissue and the boundary feature of the soft tissue corresponding to the ultrasound images or signals using signal or image processing techniques and determining the area of interest; extracting a morphological parameter of the soft tissue from the selected area of interest; according to the morphological parameter, calculating a predicted value that represents the contact state of an ultrasound probe by using at least one morphological response of the soft tissue; converting the predicted value into a time-varying indication signal to provide real-time information about the contact state between the ultrasound probe and the surface of the body and fed it back to the operator to participate in quality control during the placement of the ultrasound probe.

The technical solution adopted by this application is as follows: firstly, this application provides a method for evaluating a contact state of an ultrasound probe on the basis of soft tissue morphology, wherein, the method comprises the following steps: S11: collecting ultrasound information of morphological responses of an object under test for different contact states; S12: on the basis of a feature of soft tissue and a boundary feature of the soft tissue in the ultrasound information, recognizing a boundary position of the soft tissue in the ultrasound information; S13: on the basis of the boundary position and a preset area division condition, determining an area of interest and a sampling sub-area in the area of interest; S14: extracting a morphological parameter of the soft tissue from the selected area of interest; S15: according to the morphological parameter, calculating a predicted value that represents the contact state of an ultrasound probe by using at least one morphological response of the soft tissue; S16: converting the predicted value into a time-varying indication signal.

Wherein, the ultrasonic information in S11 includes structural imaging information of the object under test, including ultrasound signals, one-dimensional ultrasound images (A-mode and M-mode images), two-dimensional ultrasound images (B-mode images, Doppler images, elastography images), and three-dimensional ultrasound images.

Wherein, the soft tissue in S12 refers to a type of biological tissue in the object under test that has physical contact with the ultrasound probe and is capable of undergoing varying degrees of morphological changes to different contact states based on its own physical characteristics, the soft tissue includes at least one of the following: skin, subcutaneous fat, fascia, and muscles; if the ultrasonic information collected in step S11 is in the form of ultrasound signals, based on the feature of at least one soft tissue and the boundary feature of the soft tissue corresponding to the ultrasound signal, using signal processing techniques to identify the boundaries of the soft tissue, respectively.

Wherein, the area of interest in S13 includes at least one soft tissue, which is delineated by the boundary of a selected single soft tissue or the boundaries of a combination of multiple soft tissues; the sampling sub-area is obtained by dividing the selected area of interest to get at least one sub-area for collecting morphological parameters at specific spatial positions.

Wherein, the morphological parameters of the soft tissues in S14 include at least one of the following: thickness, cross-sectional area, pennation angle, and the length of muscle fiber; the area of interest contains multiple types of soft tissues, the morphological parameter is the sum of the corresponding morphological parameter values for each soft tissue; the identification of boundaries of the soft tissues and extraction of morphological parameters are performed in real-time based on each frame of the image, synchronized with the image display.

Wherein, the predicted values in S15 are conversions of morphological parameters that characterize relative pressure and relative perpendicularity of the ultrasound probes using the deformation degree of at least one soft tissue; the predicted values are directly represented by the absolute value of the morphological parameters, or calculated by mathematical statistics method using at least one real-time morphological parameter, including change rate, ratio, difference, or other measurement indicators obtained through mathematical formulas or models; translating the morphological parameters into predicted values using preset mathematical statistical methods, and the predicted values are relative values; or directly representing the predicted values by the absolute value of the morphological parameters.

Wherein, the predicted values are calculated using a combination of other parameters according to certain weights or mathematical statistical methods, the parameters include at least one of the following: physiological parameters, image feature parameters, pressure parameters, and motion parameters; when the parameter is a physiological parameter, the blood flow velocity, blood flow direction, and blood flow intensity of the object under test are extracted from the Doppler images; when the parameter is an image feature parameter, color feature parameters, texture feature parameters, shape feature parameters, and spatial relationship feature parameters are extracted from the ultrasound images; when the parameter is a pressure parameter, the pressure is collected from the pressure detection components; when the parameter is a motion parameter, the angular velocity, acceleration, velocity, and angle are collected from spatial sensors.

Wherein, the indication signal in S16 refers to real-time data of the pressure and perpendicularity between the probe and the surface of the object under test, and is used for observation and can include at least one of the following: tactile indication, auditory indication, and visual indication, the carrier of the indication signal is an indicator using to output instantaneous indication signals.

Wherein, the indicator includes software indicators and hardware indicators; software indicators include indicators displayed through color blocks, color bars, dials, scales, lights, counters, graphs, maps, or other charts and are set in any region of the virtual user interface and are displayed together with the ultrasound images; hardware indicators are indicators that provide indication signals through numerical displays, lights, images, sounds, or vibrations, and the hardware indicators are physically positioned within the ultrasound hardware apparatus at a specific location.

Wherein, further comprises: comparing the predicted values with the preset conditions, and are generating corresponding instructions based on instantaneous comparison results;

wherein, obtaining the instantaneous comparison results by evaluating the relationship between the predicted values calculated from the current frame of ultrasound images and the preset reference data, determining whether the contact state of the probe meets the preset conditions, and generating corresponding instructions based on determination results; this step is synchronized with S15.

Wherein, after comparing the predicted values with the preset conditions, and are generating corresponding instructions based on instantaneous comparison results, the following steps are also included: based on the predicted value, compensating for measured values obtained by the ultrasound equipment;

wherein, the instructions include alarm notification instructions or trigger sampling instructions; the comparison calculations performed before generating the instructions are based on real-time analysis of each frame of ultrasound images; the sampling involves collecting ultrasound images and performing functional measurements; the expression forms of alarm notifications can include at least one of the following: tactile feedback, auditory cues, and visual cues.

Wherein, the method also includes using the morphological responses of the ultrasound gel pad to evaluate the contact state of the probe, specifically including: S31: collecting the ultrasound information of the morphological response of the ultrasound gel pad and the object under test to different contact states; S32: based on the features of the corresponding ultrasound gel pad and/or soft tissue as well as the boundary features of the ultrasound gel pad and/or soft tissue in the ultrasound images or signals, using signal or image processing techniques to determine the boundary positions of the ultrasound gel pad and/or soft tissue from each frame of the ultrasound image; S33: based on the boundary of the ultrasound gel pad and/or soft tissue, as well as the preset area division condition, determining the area of interest and its sampling sub-area; S34: extracting the morphological parameters of the ultrasound gel pad and/or soft tissue in each sampling sub-area from the selected area of interest; S35: based on the morphological parameters and preset mathematical methods, calculating the predicted value using the morphological responses of the ultrasound gel pad and/or soft tissue to characterize the contact state of the probe; S36: converting the predicted value into the time-varying indication signal; wherein, the area of interest is delineated by the boundary of the ultrasound gel pad and/or soft tissue; the morphological parameters include the thickness and cross-sectional area of the ultrasound gel pad and/or soft tissue.

Wherein, the predicted values in S15 are conversions of morphological parameters that characterize relative pressure and relative perpendicularity of the ultrasound probes using the deformation degree of at least one soft tissuemorphological parameter, the calculation method is as follows, selecting thickness as a morphological parameter and thickness change rate as the predicted value; calculating the predicted value using the following change rate formula

$$Z = \frac{Y_{deformation\ state} - Y_{unloaded\ state}}{Y_{unloaded\ state}} * 100\%;$$

wherein, the predicted value z represents the degree of change in soft tissue thickness relative to the unloaded state; Y represents the overall thickness of the area of interest, in the unloaded state, the soft tissues are in a state of static relaxation, with no probe contact or minimal contact with the probe; in the deformed state, the area of interest is under the pressure of the probe; if the predicted values collected from three sampling sub-areas are Z1, Z2, and Z3, the relative

$$F = \frac{Z_1 + Z_2 + Z_3}{3} * 100\%$$

pressure value F can be calculated using the following formula                                        ; the relative pressure value F represents the percentage change in soft tissue morphology and indicates the relative pressure applied by the probe on the body surface; the relative pressure value F also represents the average pressure; the relative value of the

$$P = \frac{(|Z_1 - F| + |Z_2 - F| + |Z_3 - F|)}{3F}$$

perpendicularity P can be calculated as follows                                        ; the relative value of the perpendicularity P represents the difference in morphological response between different sampling sub-areas and can reflect the relative perpendicularity of the probe to the body surface; when P is larger, it indicates an increased difference in morphological response between the sampling sub-areas, and the tilt angle of the probe to the body surface deviates further from vertical.

Wherein, the predicted value of S15 can also be obtained by combining different parameters, which are at least one of the soft tissue morphological parameters related to ultrasound images, including the parameters involved in the weighted calculation include morphological parameter x1, the total thickness of skin and subcutaneous fat, the pressure parameter x2, the image feature parameter x3 which is the ultrasound features of soft tissues, the motion parameter x4, which is the probe attitude; the predicted value is calculated by weighted average model: suppose there are n parameters[x1, x2, x3, ..., xn] (n≥1) in the combination, and each parameter has a weight w, that is, [w1, w2, w3, ..., wn] (n≥1, wn is a constant), then the weighted average is

$$\bar{x} = \frac{w1 * x1 + w2 * x2 + ... + wn * xn}{w1 + w2 + ... + wn};$$

if based on the given weight, the predicted value after weighted processing is

$$\bar{x} = \frac{50\% * x1 + 20\% * x2 + 20\% * x3 + 10\% * x4}{50\% + 20\% + 20\% + 10\%};$$

it should be understood that the type and quantity of parameters, as well as the weights, are not limited.

Secondly, the present application provides an apparatus for the method for evaluating a contact state of an ultrasound probe on the basis of soft tissue morphology according to claim 1, wherein, comprises: an information acquisition unit for receiving an ultrasound information of an object under test; a contact state measurement unit; a contact state indication unit for converting a predicted value to a time-varying indication signal, and providing real-time contact state information between an ultrasound probe and a body surface through the indicator; the contact state measurement unit includes: a soft tissue boundary recognition unit for determining boundary positions of at least one type of soft tissue in the object under test based on the feature of at least one soft tissue and the boundary feature of the soft tissue corresponding to the ultrasound images or signals using signal or image processing techniques; an area of interest determination unit for determining an area of interest and a sampling sub-area in the area of interest on the basis of the boundary position and a preset area division condition; a soft tissue morphology quantification unit for calculating the morphological parameters of soft tissue in each sampling sub-area from the selected area of interest; a relative pressure calculation unit for converting morphological parameters into predicted values using at least one soft tissue morphology response to characterize the relative pressure of the probe according to certain mathematical statistical methods; a relative perpendicularity calculation unit for converting morphological parameters into predicted values that use at least one soft tissue morphology response to characterize the relative perpendicularity of the probe according to certain mathematical statistical methods.

Wherein, the contact state measurement unit includes an added pressure compensation unit and a perpendicularity compensation unit which are respectively used to compensate for the measurement values obtained by the ultrasound equipment based on the relative pressure information and relative perpendicularity information calculated by the relative pressure calculation unit and the relative perpendicularity calculation unit.

The beneficial effects of this application are as follows:

This method has the advantages of quantitative evaluation, ease of use, and low professional requirements for users. As an auxiliary function, this application only requires software modification without any hardware modifications to assess the contact state of an ultrasound probe. This application can be extended to conventional ultrasound imaging devices, significantly reducing costs and promoting standardized operations. It overcomes the shortcomings of strong subjectivity, lack of quantitative reference standards, and low reliability in traditional manual operations. It also overcomes the drawbacks of hardware modification and low generalizability to different populations in existing engineering approaches.

## DESCRIPTION OF THE DRAWINGS

Figure 1 is a schematic diagram illustrating the method for evaluating a contact state of an ultrasound probe on the basis of soft tissue morphology according to the first embodiment of the present application.

Figure 2 is a schematic diagram illustrating the method for evaluating a contact state of an ultrasound probe on the basis of soft tissue morphology according to the second embodiment of the present application.

Figure 3 is a schematic diagram illustrating the method for evaluating a contact state of an ultrasound probe on the basis of soft tissue morphology according to the third embodiment of the present application.

Figure 4 is a schematic diagram illustrating the method for evaluating a contact state of an ultrasound probe on the basis of soft tissue morphology according to the fourth embodiment of the present application.

Figure 5 is a schematic diagram illustrating the calculation of relative pressure and relative perpendicularity of the probe in the aforementioned embodiments of the present application.

Figure 6 is a schematic diagram illustrating the calculation of predicted values using different combinations of parameters in an embodiment of the present application.

Figure 7 is a schematic diagram illustrating the involvement of an ultrasound gel pad in evaluating the contact state of an ultrasound probe in the third embodiment of the present application.

Figure 8 is a schematic diagram illustrating the structural configuration of an apparatus for the method for evaluating a contact state of an ultrasound probe on the basis of soft tissue morphology according to the present application.

Figure 9 is a schematic diagram illustrating the structural configuration of an apparatus for the method for evaluating a contact state of an ultrasound probe on the basis of soft tissue morphology according to the present application.

Figure 10 is a schematic diagram illustrating the structural configuration of an application embodiment (applied to quality control of liver elasticity detection) of the method for evaluating a contact state of an ultrasound probe on the basis of soft tissue morphology according to the present application.

Figure 11 is a schematic diagram illustrating the structural configuration of another application embodiment (applied to quality control of musculoskeletal ultrasound quantitative measurement) of the method for evaluating a contact state of an ultrasound probe on the basis of soft tissue morphology according to the present application.

## DETAILED DESCRIPTION OF THE PREFERRED EMBODIMENT

It should be noted that, unless otherwise conflicting, the embodiments and features described in this application can be combined with each other.

## The first embodiment

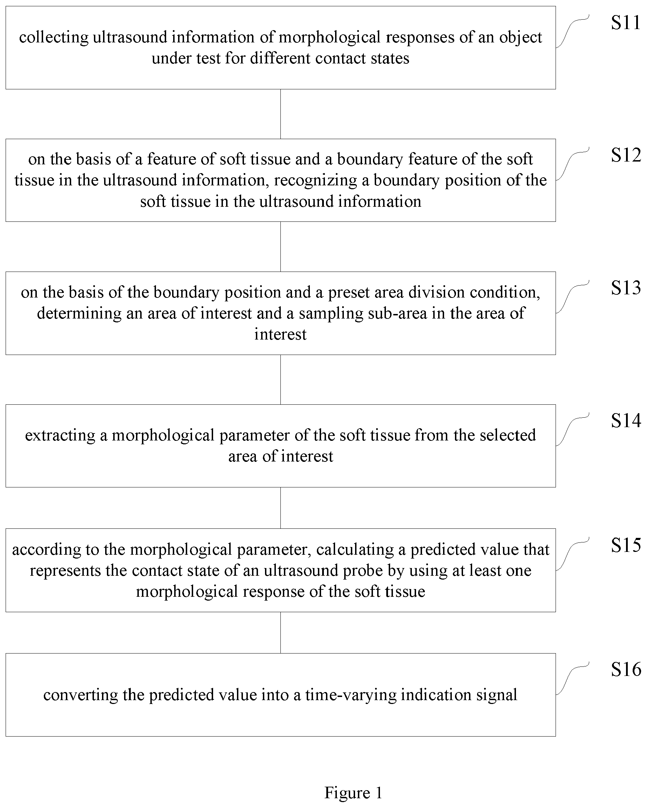

Please refer to Figure 1, which is a schematic diagram illustrating the method for evaluating a contact state of an ultrasound probe on the basis of soft tissue morphology according to the first embodiment of the present application. As shown in Figure 1, the method comprises the following steps:

S11: collecting ultrasound information of morphological responses of an object under test for different contact states;

In step S11, the term "object under test" refers to any organ of the human or animal body that can be imaged by ultrasound. Correspondingly, the biological tissues that can be displayed in ultrasound images or signals include, but are not limited to, anatomical structures of soft tissues such as skin, subcutaneous fat, fascia, ligaments, blood vessels, nerves, and muscles, as well as anatomical structures of non-soft tissues such as bones and specific organs in their vicinity.

The contact state refers to the pressure and/or perpendicularity between the ultrasound probe and the surface of the object under test. When the contact state between the ultrasound probe and the object under test changes, the object under test undergoes different deformation states.

Wherein, the ultrasonic information includes structural imaging information of the object under test, including ultrasound signals, one-dimensional ultrasound images (A-mode and M-mode images), two-dimensional ultrasound images (B-mode images, Doppler images, elastography images), and three-dimensional ultrasound images. Non-exhaustively, one-dimensional ultrasound signals and A-mode ultrasound images can reflect the one-dimensional morphology of soft tissues in the object under test. M-mode ultrasound images can provide dynamic information about the one-dimensional morphology of soft tissues in the object under test. B-mode ultrasound images can reflect the two-dimensional morphology of soft tissues in the object under test. Three-dimensional ultrasound images can reflect the three-dimensional spatial morphology of soft tissues in the object under test.

In step S11, once the object under test is identified, the ultrasound probe is placed on the surface of the object's skin. During the contact process between the probe and the selected position, it can cause a certain degree of deformation in a specific type of soft tissue beneath the contact point. Simultaneously, the ultrasound transducer emits ultrasound waves and receives the echo signals reflected by the object under test, allowing real-time acquisition of ultrasound information from the object under test.

Specifically, before initiating image acquisition or functional measurements, the ultrasound equipment undergoes the following process: the ultrasound probe is made to make contact with the surface of the skin, and then the force is applied and the inclination angle is continuously adjusted in real-time to ensure good contact between the probe and the body surface, until it is stabilized within an appropriate contact state or range. During the placement process of the probe, an ultrasound image sequence is acquired, which captures the response of the morphology of the object under test to different contact states. Common methods of probe placement include manual handheld, adhesive tape, straps, fixation devices, and adhesive patches.

S12: on the basis of a feature of soft tissue and a boundary feature of the soft tissue in the ultrasound information, recognizing a boundary position of the soft tissue in the ultrasound information;

In step S12, the soft tissue includes at least one of the following: skin, subcutaneous fat, fascia, and muscles.

It should be noted that the soft tissue in S12 refers to a type of biological tissue in the object under test that has physical contact with the ultrasound probe and is capable of undergoing varying degrees of morphological changes to different contact states based on its own physical characteristics. In ultrasound imaging, the soft tissue appears as tissue structures, locations, adjacent structures, and hierarchical relationships within relatively superficial regions that can be displayed by the spatial resolution of existing ultrasound imaging. The morphological response is manifested by the variations in imaging information of various layers of soft tissues, such as the skin layer, subcutaneous fat layer, fascia layer, and muscle layer.

Specifically, through signal and image processing technology, the ultrasonic image sequence is preprocessed by digitization, Geometric transformation, image enhancement, etc. If the ultrasonic information collected in step S11 is in the form of ultrasound signals, based on the feature of at least one soft tissue and the boundary feature of the soft tissue corresponding to the ultrasound signal, using signal processing techniques to identify the boundaries of the soft tissue, respectively; if the ultrasonic information collected in step S11 is in the form of ultrasound images, based on the feature of at least one soft tissue and the boundary feature of the soft tissue corresponding to the ultrasound images, using image processing techniques. For example, it is possible to determine the boundary of a certain region as the boundary of the skin area, the boundary of another region as the boundary of the subcutaneous fat area, the boundary of another region as the boundary of the fascia area, and the boundary of another region as the boundary of the muscle area.

S13: on the basis of the boundary position and a preset area division condition, determining an area of interest and a sampling sub-area in the area of interest;

Specially, the area of interest in S13 includes at least one soft tissue, which is delineated by the boundary of a selected single soft tissue or the boundaries of a combination of multiple soft tissues. A region is defined for further processing. From an anatomical perspective, the area of interest refers to a specific segment of a single type of soft tissue or a specific segment of a composite of multiple soft tissues. From an imaging perspective, the area of interest refers to a specific imaging section of a single type of soft tissue or a combination of multiple soft tissues.

The region division is based on pre-defined criteria for region division. According to these criteria, the area of interest is divided into at least one sampling sub-area. These sampling sub-areas are used to collect morphological parameters at specific spatial positions.

S14: extracting a morphological parameter of the soft tissue from the selected area of interest;

The morphological parameters of the soft tissues in S14 include at least one of the following: thickness, cross-sectional area, pennation angle, and the length of muscle fiber. Based on the selected area of interest, at least one morphological parameter of the soft tissue in each sampling sub-area is obtained.

The morphological parameter is understood as the representation of the response and the effects of soft tissues in the object under test produced under different probe contact states. In practical implementation, if the image to be processed is a one-dimensional ultrasound image (such as A-mode and M-mode images), the morphological parameter can be thickness. If the image to be processed is a two-dimensional ultrasound image (such as B-mode image), the morphological parameters can include thickness, cross-sectional area, feathering angle, or muscle fiber length. If the image to be processed is a three-dimensional ultrasound image, the morphological parameter is volume. In the case of the area of interest containing multiple types of soft tissues, it should be understood that the final determined morphological parameter is the sum of the corresponding morphological parameter values for each type of soft tissue.

S15: according to the morphological parameter, calculating a predicted value that represents the contact state of an ultrasound probe by using at least one morphological response of the soft tissue;

In step S15, the morphological parameter is transformed into a predicted value based on a pre-defined mathematical statistical method. This mathematical statistical method can be abstracted as a mathematical formula, where the morphological parameter serves as the input parameter of the formula, and the predicted value is the output value of the formula.

The predicted value can indeed be an absolute value. It can be the absolute value of the morphological parameter itself, or it can be obtained by calculating at least one morphological parameter using a mathematical statistical method. For example, the predicted value can be a multiple of the absolute value of the morphological parameter, or it can be the absolute value of the morphological parameter plus a constant value.

Preferably, the predicted value is a relative value, representing the predicted values of relative pressure and relative perpendicularity of the probe. Preferably, the predicted value can be normalized, meaning the predicted value falls within the range of 0 to 1. Non-limiting implementation methods include measures such as rate of change, ratio, difference, or other measurement indicators obtained through mathematical formulas or models.

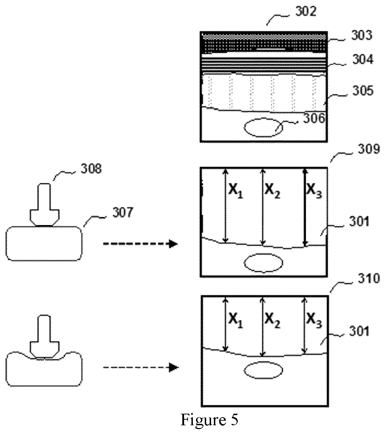

Please refer to Figure 5, which is a schematic diagram illustrating the calculation of relative pressure and relative perpendicularity of the probe. Figure 5 represents a structural diagram of an embodiment of step S15 of Figure 1. The predicted value is based on the rate of change of the morphological parameters relative to the unloaded state. The morphological parameters in the unloaded state correspond to the morphological parameters of the soft tissue obtained from the ultrasound information when the ultrasound probe is in the unloaded state. It can be understood that when calculating the rate of change, the morphological parameters in the deformed state and the morphological parameters in the unloaded state should correspond to the soft tissue in the same region in the deformed state and the unloaded state, respectively.

In Figure 5, 302 represents the region of the B-mode ultrasound image, which includes the area of interest 301 and a specific organ 306 located deeper. The area of interest 301 is a composite structure within the B-mode ultrasound image 302, consisting of the skin layer 303, subcutaneous fat layer 304, and a superficial muscle layer 305 (located at the most superficial position, below the subcutaneous fat layer).

The area of interest 301 includes several sampling sub-areas arranged along the longitudinal axis of the area of interest, namely $X_1$, $X_2$, $X_3$. The morphological parameters extracted from each sampling sub-area are denoted as $Y_1$, $Y_2$, $Y_3$, which in this embodiment represent the thickness. In this embodiment, it can be understood that $Y_n$ represents the overall thickness of the area of interest, $Y_n = Y_{n1} + Y_{n2} + Y_{n3}$, where n is the index of the sampling sub-area and n>=1.

In Figure 5, 307 represents the object under test, and 308 represents the probe. Force is applied to the probe 308, causing the area of interest 301 to be changed from the unloaded state (t0) 309 to the deformed state 310 (t1). It is easy to be observed that $Y_n$ undergoes a significant change at this point. For example, let's denote the predicted value Z as the rate of change of thickness, which characterizes the contact state of the probe.

$$Z = \frac{Y_{deformation\ state} - Y_{unloaded\ state}}{Y_{unloaded\ state}} * 100\%.$$

If the predicted values collected from three sampling sub-areas are Z1, Z2, and Z3, the relative pressure value F can be calculated using the following formula $F = \frac{Z_1 + Z_2 + Z_3}{3} * 100\%$ ; the relative pressure value F represents the percentage change in soft tissue morphology and indicates the relative pressure applied by the probe on the body surface; the relative pressure value F also represents the average pressure.

The relative value of the perpendicularity P can be calculated as follows $P = \frac{(|Z_1 - F| + |Z_2 - F| + |Z_3 - F|)}{3F}$ ; the relative value of the perpendicularity P represents the difference in morphological response between different sampling sub-areas and can reflect the relative perpendicularity of the probe to the body surface. The relative perpendicularity is based on a selected two-dimensional plane. When P is small, it indicates that the morphological responses of the sampling sub-areas are similar. This implies that the force distribution of the probe is balanced, and the probe is close to being perpendicular to the body surface. When P is larger, it indicates an increased difference in morphological response between the sampling sub-areas, and the tilt angle of the probe to the body surface deviates further from vertical.

Furthermore, this predicted value can also be obtained through a combination of different parameters. Specifically, it involves combining parameter information from different dimensions and assigning certain weights or using various mathematical and statistical models to calculate a comprehensive score. In an ideal scenario, the dimensions are mutually complementary and complete.

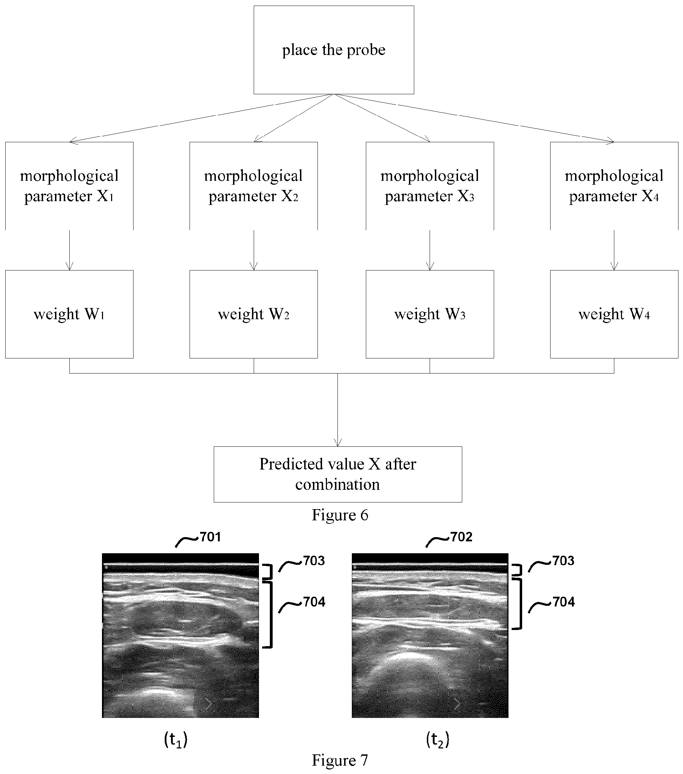

Please refer to Figure 6, which is a structural diagram of another embodiment of step S15 of Figure 1. As shown in Figure 6, the predicted value Z is calculated by multiplying the parameter information from different dimensions by the corresponding weights assigned to each parameter. At least one of these parameters corresponds to the morphological parameters obtained in step S14, which are derived from ultrasound information.

Other non-restrictive parameters include physiological parameters, image feature parameters, pressure parameters, and motion parameters, among others.

The physiological parameter refers to the hemodynamic characteristics measured using the Doppler effect of ultrasound. It is associated with the physiological activity of the selected soft tissue and is understood as the blood flow velocity, direction, intensity, etc., acquired through Doppler imaging in a non-invasive manner.

The image feature parameters refer to the characteristics of the soft tissue and its boundaries extracted using digital image processing techniques. They are used to identify the relative positional information of the target tissue and ensure consistency in the placement of the probe in the relative space. Another implementation is to evaluate the quality of ultrasound images using image processing techniques, including resolution, artifacts, and noise distribution. For example, if there is insufficient coupling agent, the decrease in image quality can also reflect the poor contact status of the probe to some extent. In a non-restrictive manner, it is understood as the color features, texture features, shape features, spatial relationship features, etc., acquired through ultrasound image acquisition.

The pressure parameter refers to the mechanical quantity recorded using external devices such as piezoelectric force sensors, strain force sensors, or a combination of springs and position sensors. It directly evaluates the physical contact between the probe and the body surface. In a non-restrictive manner, it is understood as the pressure acquired through pressure detection components.

The motion parameter refers to the dynamic motion data recorded using external devices such as accelerometers, gyroscopes, or inertial measurement units. It is used to calculate the position and motion attitude of the ultrasound probe in three-dimensional space, ensuring the physical verticality of the probe in the relative space. In a non-restrictive manner, it is understood as the angular velocity, acceleration, velocity, angle, etc., acquired through spatial sensors. It should be noted that this embodiment does not limit the type and quantity of model combinations or the type and quantity of parameters.

The predicted value is calculated by weighted average model: suppose there are n parameters [x1, x2, x3, ..., xn] (n≥1) in the combination, and each parameter has a weight w, that is, [w1, w2, w3, ..., wn] (n≥1, wn is a constant),

$$x = \frac{w_1 * x_1 + w_1 * x_1 + \dots w_n * x_n}{w_1 + \dots + w_n}$$

then the weighted average is . Specifically, please refer to Figure 6. The parameters involved in the weighted calculation are morphological parameter $x_1$ (such as total thickness of skin and subcutaneous fat), pressure parameter $x_2$ (such as pressure value), image feature parameter $x_3$ (such as ultrasound features of soft tissue), and motion parameter $x_4$ (such as probe posture). If based on the given weights, the weighted predicted value

$$x = \frac{50\% * x_1 + 20\% * x_2 + 20\% * x_3 + 10\% * x_4}{50\% + 20\% + 20\% + 10\%}$$

after processing is . It should be understood that the type and quantity of parameters, as well as the weights, are not restrictive.

S16: converting the predicted value into a time-varying indication signal.

The indication signal refers to real-time data of the pressure and perpendicularity between the probe and the surface of the object under test, and is used for observation and can include at least one of the following: tactile indication, auditory indication, and visual indication, the carrier of the indication signal is an indicator using to output instantaneous indication signals.

The indicator includes software indicators and hardware indicators; software indicators include indicators displayed through color blocks, color bars, dials, scales, lights, counters, graphs, maps, or other charts and are set in any region of the virtual user interface and are displayed together with the ultrasound images.

Hardware indicators are indicators that provide indication signals through numerical displays, lights, images, sounds, or vibrations, and the hardware indicators are physically positioned within the ultrasound hardware apparatus at a specific location。

In step S16, the predicted value is converted into a form that can be easily understood by ordinary users. The instructions should be simple and easy to understand, without requiring advanced anatomical knowledge or medical imaging interpretation skills.

In this embodiment, ultrasound information is collected to capture the morphological response of the object under test in its current contact state. The morphological parameters corresponding to the soft tissues in this ultrasound information are obtained. Based on these acquired morphological parameters, a predicted value is calculated to characterize the contact state of the ultrasound probe. This predicted value can reflect the probe's contact state, including the force applied and the perpendicularity of the probe. On the basis of an existing ultrasound imaging apparatus, and by using software technical means, an apparatus for evaluating a contact state of an ultrasound probe on the basis of soft tissue morphology can accurately calculate the contact state of the ultrasound probe without needing to change the hardware, thereby facilitating the operation of the apparatus by an operator.

Furthermore, this predicted value can be a relative value, ensuring comparability across different body types and anatomical structures in the tested population. By creating a measurement standard that adapts to individual differences and is applicable to different anatomical structures, the readings can be comparable among different populations or different body parts. In the methods that rely on force sensors to assess probe contact pressure, if the predicted value falls within a predetermined range (such as 10N to 20N) of a certain absolute pressure value, this reference range may be suitable for obese participants but may not be applicable to non-obese participants.

The second embodiment:

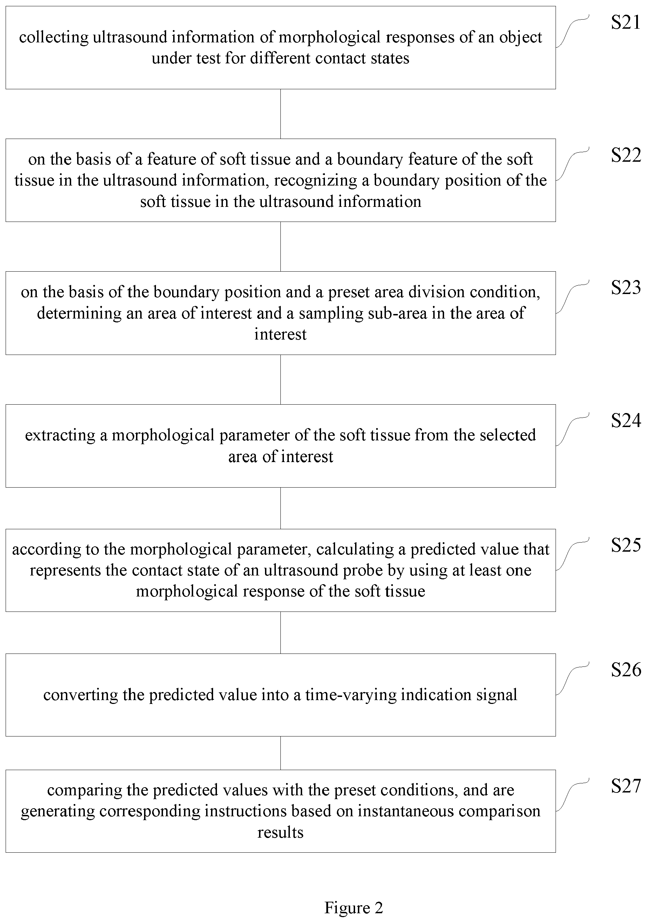

Please refer to Figure 2. Figure 2 is a schematic diagram illustrating the method for evaluating a contact state of an ultrasound probe on the basis of soft tissue morphology according to the second embodiment of the present application. As shown in Figure 2, this method differs from the method of the first embodiment in that it further includes step S27: comparing the predicted values with the preset conditions and generating corresponding instructions based on the instantaneous comparison result. Step S27 is performed synchronously with step S25.

The preset conditions can be a preset value (i.e., a single value K) or a range of preset values (i.e., composed of a certain range of values $K \pm K_1$). In the specific implementation, the instantaneous comparison result is obtained based on the relationship between the predicted value calculated from the current frame ultrasound image and the preset conditions. This result is used to determine whether the contact state of the probe meets the preset conditions, and corresponding instructions are generated based on the determination result.

The comparison calculation before generating instructions is performed in real-time based on each frame of the ultrasound image.

The instruction includes alarm notification instructions and/or trigger operation instructions. The alarm notification instruction is used to alert the operator to correct any poor or abnormal conditions regarding the probe contact or to remind the operator to manually sample the object under test. For example, if the predicted value reaches a certain threshold range, it indicates that manual sampling can be performed on the object under test. Therefore, when the operator receives a specific alarm notification instruction, they can proceed with the sampling operation. The alarm notification instruction can be conveyed to the operator through auditory, tactile, and visual cues.

The trigger operation instruction is used to perform automatic sampling on the object under test. Sampling includes ultrasound image acquisition and functional measurements. Specifically, when the predicted value reaches a certain threshold range, the ultrasound device is directly commanded to perform automatic sampling on the object under test. The advantage of using automatic sampling is that it eliminates the need for manual judgment, thereby improving the accuracy of triggering ultrasound detection.

It should be noted that the sampling mentioned here refers to triggering ultrasound image acquisition and functional measurements on the object being measured, such as transient elastography and pulse wave Doppler blood flow measurements.

**The third embodiment**

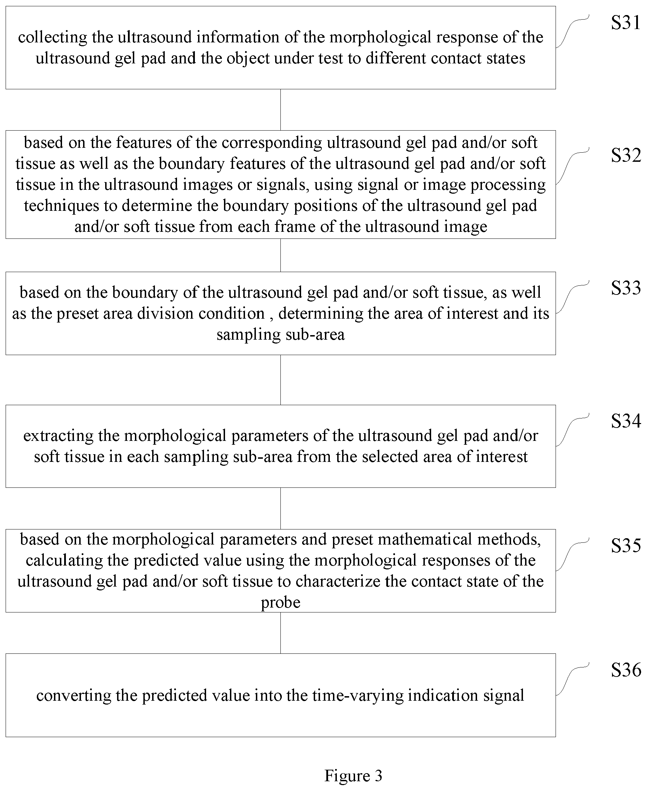

Please refer to Figure 3. Figure 3 is a schematic diagram illustrating the method for evaluating a contact state of an ultrasound probe on the basis of soft tissue morphology according to the third embodiment of the present application. The application scenario of this embodiment is as follows: when the surface of the object under test is uneven or the object under test is located close to the skin surface, the use of an ultrasound gel pad can not only effectively improve the fit between the ultrasound probe and the skin but also enhance near-field resolution to improve imaging quality. In the method described in this example, before implementing the method, the operator first places the ultrasound gel pad on the surface of the object under test and positions the ultrasound probe on the surface of the ultrasound gel pad above the object under test. As shown in Figure 3, the method includes the following steps:

S31: collecting the ultrasound information of the morphological response of the ultrasound gel pad and the object under test to different contact states;

The difference between step S31 and step S11 in the first embodiment is that in step S31, the ultrasound information includes not only the ultrasound information of the object under test but also the ultrasound information of the ultrasound gel pad.

S32: based on the features of the corresponding ultrasound gel pad and/or soft tissue as well as the boundary features of the ultrasound gel pad and/or soft tissue in the ultrasound images or signals, using signal or image processing techniques to determine the boundary positions of the ultrasound gel pad and/or soft tissue from each frame of the ultrasound image;

The difference between step S32 and step S12 in the first embodiment is that in step S12, the obtained morphological parameters correspond to the soft tissue in the ultrasound information. In contrast, in step S32, the obtained morphological parameters correspond to either the ultrasound gel pad in the ultrasound information, or the soft tissue in the ultrasound information, or a combination of the soft tissue and the ultrasound gel pad in the ultrasound information.

S33: based on the boundary of the ultrasound gel pad and/or soft tissue, as well as the preset area division condition, determining the area of interest and its sampling sub-area;

The difference between step S33 and step S13 in the first embodiment is that in step S33, the determined area of interest is the area of interest corresponding to the ultrasound gel pad, or includes the area of interest corresponding to soft tissue and the area of interest corresponding to ultrasound gel pad.

The area of interest is defined by the boundaries of the ultrasound gel pad and/or soft tissue. The morphological parameters include the thickness and cross-sectional area of the ultrasound gel pad and/or soft tissue.

S34: extracting the morphological parameters of the ultrasound gel pad and/or soft tissue in each sampling sub-area from the selected area of interest;

The method for extracting morphological parameters is the same as step S14 in the first embodiment.

S35: based on the morphological parameters and preset mathematical methods, calculating the predicted value using the morphological responses of the ultrasound gel pad and/or soft tissue to characterize the contact state of the probe;

Step S35 shares the same calculation method as step S15 in the first embodiment. The difference lies in the fact that the morphological parameter in step S35 can be either the morphological parameter corresponding to the ultrasound gel pad or a combination of morphological parameters including both the soft tissue and the ultrasound gel pad.

Based on the provided information, the calculation method of this embodiment can be illustrated with reference to Figure 7. In Figure 7, 703 represents the morphological parameter (thickness) corresponding to the ultrasound gel pad, and 704 represents the morphological parameter (thickness) corresponding to the soft tissue. The B-mode ultrasound image 701 at time t1 corresponds to the ultrasound information of the object under test in the unloaded state of the ultrasound probe. The B-mode ultrasound image 702 at time t2 corresponds to the ultrasound information of the object under test in a certain loaded state of the ultrasound probe. From the figure, it can be observed that both the morphological parameter 703 corresponding to the ultrasound gel pad and the morphological parameter 704 corresponding to the soft tissue decrease from time t1 to time t2. Based on the rate of change of the morphological parameter 703 corresponding to the ultrasound gel pad at time t2, or the rate of change of the morphological parameter 704 corresponding to the soft tissue, or a combination of both, a predicted value representing the probe contact state can be calculated. In other embodiment, the state corresponding to time t1 can be a deformation state under a certain loaded condition rather than the unloaded state.

S36: converting the predicted value into the time-varying indication signal.

In this embodiment, the difference from the first embodiment lies in the ability to place a ultrasound gel pad between the object under test and the probe when the surface of the object is uneven or when the object is positioned shallowly. Based on the morphological parameter corresponding to the ultrasound gel pad, a predicted value representing the ultrasound probe contact state can be calculated.

**The fourth embodiment:**

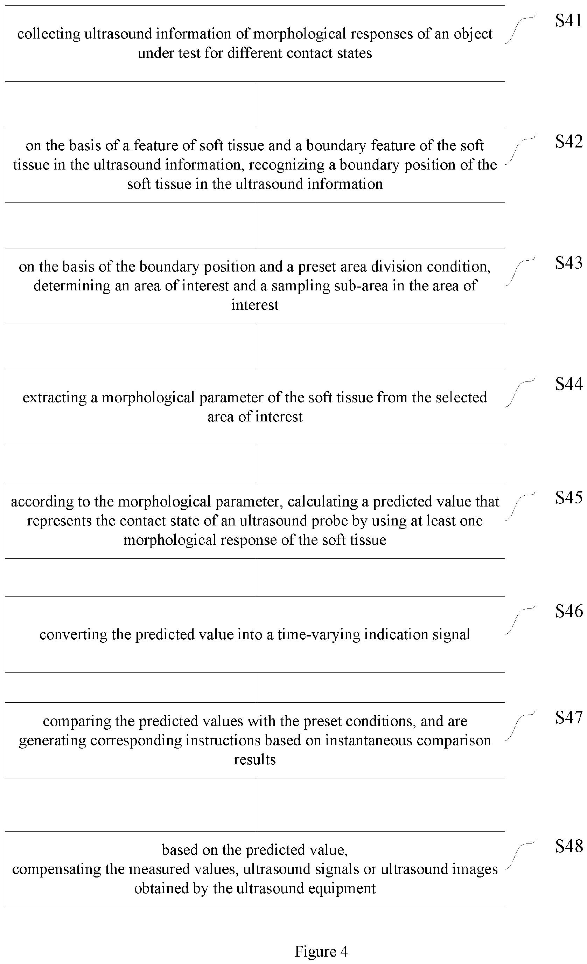

Please refer to Figure 4. Figure 4 is a schematic diagram illustrating the method for evaluating a contact state of an ultrasound probe on the basis of soft tissue morphology according to the fourth embodiment of the present application. As shown in Figure 4, the difference between this method and the method in the second embodiment is that after step S47, it includes step S48: compensating the measured values obtained by the ultrasound equipment based on the predicted value. The specific functional measurement depends on the hardware and algorithms of the existing ultrasound equipment. In non-limiting embodiments, the measured values can be liver elasticity values obtained by an instantaneous elastography device.

It can be understood that the compensation method can involve a mathematical formula that incorporates empirical values. The predicted value can be an absolute value or a relative value. For example, the relative value can represent the predicted value of the probe's relative pressure and the predicted value of the probe's relative perpendicularity.

In this embodiment, the measured values or ultrasound data can be compensated based on the predicted value. This compensation can effectively eliminate the differences between the measured values, ultrasound data, and the actual values.

**The fifth embodiment:**

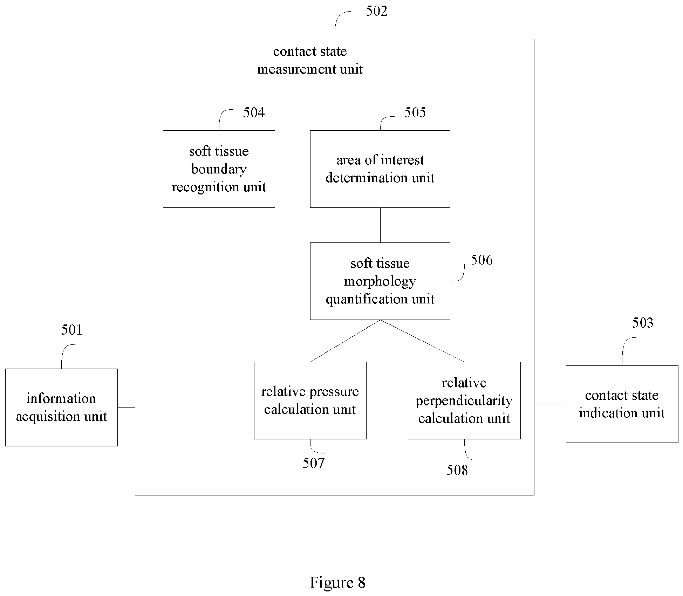

Please refer to Figure 8. Figure 8 is a schematic diagram illustrating the structural configuration of an apparatus for the method for evaluating a contact state of an ultrasound probe on the basis of soft tissue morphology according to the present application. As shown in Figure 8, the apparatus includes an information acquisition unit 501, a contact state measurement unit 502, and a contact state indication unit 503.

The contact state measurement unit 502 includes a soft tissue boundary recognition unit 504, an area of interest determination unit 505, a soft tissue morphology quantification unit 506, a relative pressure calculation unit 507, a relative perpendicularity calculation unit 508. The contact state measurement unit 502 includes a soft tissue boundary recognition unit 504, an area of interest determination unit 505, a soft tissue morphology quantification unit 506, a relative pressure calculation unit 507, a relative perpendicularity calculation unit 508.

Specifically, the information acquisition unit 501 is for receiving an ultrasound information of an object under test. Wherein, the ultrasound information is the ultrasound signal, one-dimensional ultrasound images, two-dimensional ultrasound images or three-dimensional ultrasound images including structural imaging information of the soft tissue. The contact state measurement unit 502 is composed of the soft tissue boundary recognition unit 504, the area of interest determination unit 505, the soft tissue morphology quantification unit 506, the relative pressure calculation unit 507 and the relative perpendicularity calculation unit 508. Its function is to quantitatively analyze the geometric structure of soft tissues in ultrasound image sequences. This is achieved by utilizing various signal or image processing techniques such as recognition, focusing, matching, segmentation, and feature calculation on each frame of the ultrasound image sequence. The soft tissue boundary recognition unit 504 is for determining boundary positions of at least one type of soft tissue in the object under test based on the feature of at least one soft tissue and the boundary feature of the soft tissue corresponding to the ultrasound images or signals using signal or image processing techniques. The area of interest determination unit 505 is for determining an area of interest and a sampling sub-area in the area of interest on the basis of the boundary position and a preset area division condition. The soft tissue morphology quantification unit 505 is for calculating the morphological parameters of soft tissue in each sampling sub-area from the selected area of interest. The relative pressure calculation unit 507 is for converting morphological parameters into predicted values using at least one soft tissue morphology response to characterize the relative pressure of the probe according to certain mathematical statistical methods. The relative perpendicularity calculation unit 508 is for converting morphological parameters into predicted values that use at least one soft tissue morphology response to characterize the relative perpendicularity of the probe according to certain mathematical statistical methods. The contact state indication unit 503 is for converting a predicted value to a time-varying indication signal, and providing real-time contact state information between an ultrasound probe and a body surface through the software indicators or hardware indicators.

Preferably, the system composed of the aforementioned units can be integrated as an add-on component into the software of existing ultrasound equipment, providing a new functionality for real-time monitoring of the contact state of the ultrasound probe. It is worth mentioning that the integration process does not require any hardware modifications. In terms of quality control for probe placement, this system offers a low-cost solution with high accessibility.

**The sixth embodiment:**

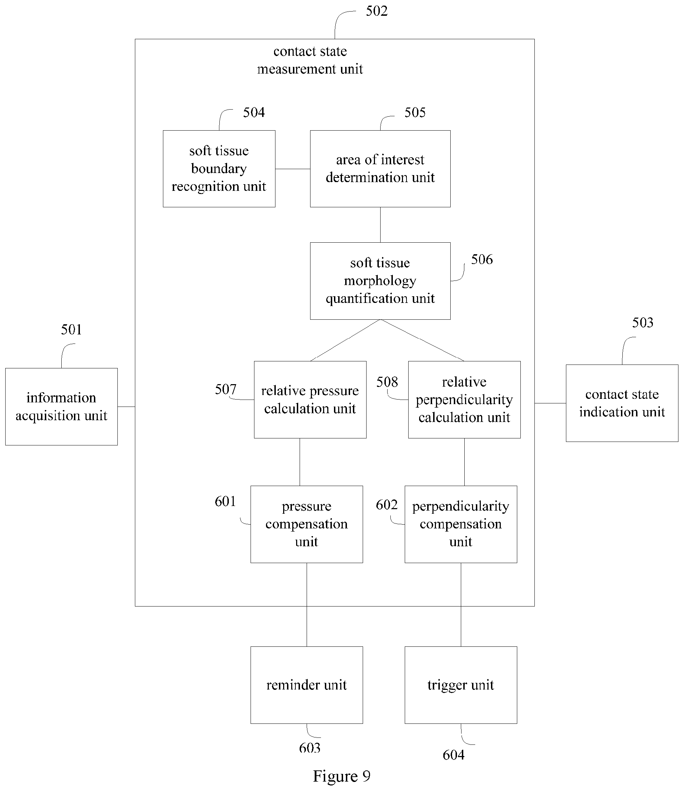

Please refer to Figure 9. Figure 9 is a schematic diagram illustrating the structural configuration of an apparatus for the method for evaluating a contact state of an ultrasound probe on the basis of soft tissue morphology according to the present application. The difference between Figure 9 and Figure 8 is that the contact state measurement unit 502 further includes a pressure compensation unit 601, a perpendicularity compensation unit 602, a reminder unit 603 and a trigger unit 604.

The pressure compensation unit 601 is responsible for compensating the measured values based on the relative pressure information calculated by the relative pressure calculation unit 507. The perpendicularity compensation unit 602 compensates the measured values based on the relative perpendicularity information calculated by the relative perpendicularity calculation unit 508. It is important to note that the measured values are understood to be obtained from existing ultrasound equipment, and the specific measurement functionality depends on the hardware and algorithms of the existing ultrasound equipment. An exemplary non-limiting implementation is compensating the liver elasticity values measured by an instantaneous elasticity detection device. The reminder unit 603 is responsible for determining whether the current probe contact state meets the preset conditions and generating alarm prompt to the operator. The trigger unit 604 is used to send a trigger command to the existing ultrasound equipment, instructing it to perform image acquisition and functional measurements on the object under test.

**The seventh embodiment:**

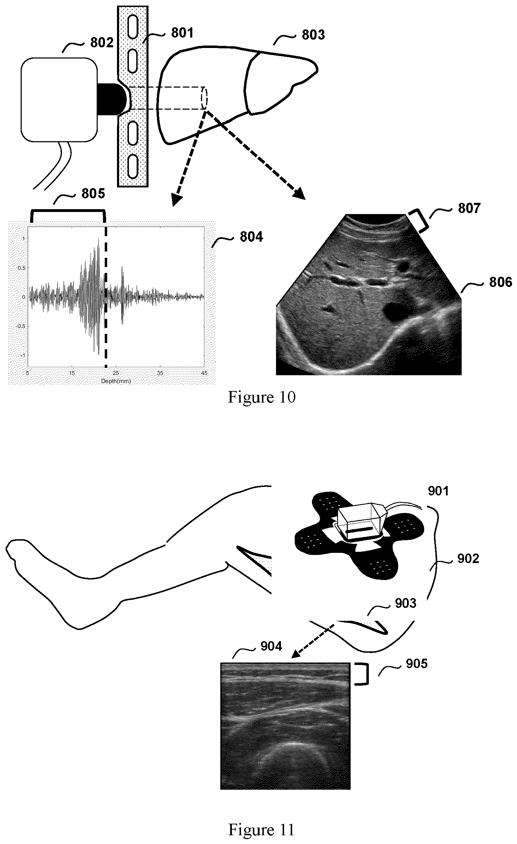

Please refer to Figure 10. Figure 10 is a schematic diagram illustrating the structural configuration of an application embodiment (applied to quality control of liver elasticity detection) of the method for evaluating a contact state of an ultrasound probe on the basis of soft tissue morphology according to the present application.

This embodiment describes the application scenario of instant single-point measurement. Taking the example of ultrasound transient elastography, in the process of measuring liver elasticity, the operator needs to place the probe on the surface of the intercostal space and increase the contact pressure to create an acoustic window that allows ultrasound waves to pass smoothly. By applying an internal (self-generated) or external stimulus to the liver tissue and using ultrasound waves to track the propagation of shear waves, an instant elasticity reading is calculated. Inappropriate probe pressure not only affects the ultrasound imaging quality of the liver but also reduces the success rate and reliability of elasticity measurements.

Different contact states between the probe and the skin surface of the intercostal space can change the numerical value of the skin-to-liver capsule distance (SCD). Anatomically, SCD includes various soft tissues such as skin, muscles, and subcutaneous fat. In ultrasound imaging, SCD refers to the straight-line distance from the skin to the liver capsule, including various imaging anatomical structures such as the skin layer, fat layer, intercostal muscle layer, and external oblique muscle layer. It should be understood that the types and quantities of soft tissues included in SCD are not limited. In this embodiment, a method is provided to assess the contact state of an ultrasound probe based on SCD. A specific intercostal space 801 can serve as a natural acoustic window, and an ultrasound probe 802 is used to image biological tissues such as the liver 803. If a one-dimensional ultrasound signal 804 of the liver is acquired, the ultrasound signal before the dashed line represents the signal of SCD 805, and the ultrasound signal after the dashed line represents the signal of the liver tissue. The variation in SCD 805 can be used to characterize the probe's contact state. If a two-dimensional B-mode ultrasound image 806 of the liver is acquired, the cross-sectional area 807 of SCD within a specific section can be used to characterize the probe's contact state.

**The eighth embodiment:**

Please refer to Figure 11. Figure 11 is a schematic diagram illustrating the structural configuration of another application embodiment (applied to quality control of musculoskeletal ultrasound quantitative measurement) of the method for evaluating a contact state of an ultrasound probe on the basis of soft tissue morphology according to the present application.

This embodiment describes the method applicable to continuous monitoring scenarios. In the context of ultrasound imaging for assessing dynamic muscle movements, an ultrasound probe 901 needs to be placed or fixed on the skin surface of a specific anatomical site 902 for an extended period to image biological tissues such as muscles 903. If a two-dimensional B-mode ultrasound image 904 of the muscle is acquired, the position information of the overall thickness distribution data of the soft tissues within a specific section can be used to characterize the probe's contact state. This overall thickness includes the combined thickness 905 of the skin layer and subcutaneous fat layer. Indeed, an inappropriate probe contact state can have a negative impact on the effectiveness of the application. This method can be used for long-term monitoring of muscle contractions to detect instances of poor contact, loosening, or detachment of the probe. It is worth noting that this embodiment is not limited to diagnostic ultrasound and can also be applied to therapeutic ultrasound equipment used in physical therapy.

In summary, the present application provides a method and apparatus for evaluating a contact state of an ultrasound probe on the basis of soft tissue morphology. Compared to conventional ultrasound imaging methods, it offers the following technical advantages:

1. Quality control for probe placement: by quantifying pressure and perpendicularity, the operator can actively control the contact state between the ultrasound probe and the object under test in real-time, ensuring that the ultrasound equipment is operated within the optimal range. This method is applicable for both single measurements and continuous monitoring.

2. Assessment of probe contact state using intrinsic information: the contact state is determined based on the structural information of the soft tissues at the probe location, directly reflecting the true impact of probe placement on the object under test. There is no need to rely on external devices to estimate the contact state.

3. Improved repeatability: for the same operator during different testing sessions, this application ensures consistency in applied pressure and perpendicularity when the probe is repositioned, minimizing measurement errors.

4. Improved reproducibility: for different operators, this application ensures consistency in applied pressure and perpendicularity when the probe is repositioned, minimizing measurement errors.

5. Low requirements for operator experience and expertise: this application transforms complex ultrasound image data into an easily understandable form. The guidance process is simple and clear, eliminating the need for in-depth anatomical knowledge and medical image interpretation skills.

6. Individualized probe placement: the use of relative values for guidance allows for customization to accommodate individuals with different body types and anatomical structures, ensuring comparable results.

7. Flexibility in usage: this application can be implemented by incorporating specific algorithms into existing software, without the need for hardware modifications. It enables the evaluation of probe contact state using conventional ultrasound imaging apparatus.

8. Low-cost solution suitable for various ultrasound devices: this application provides a cost-effective solution that can be applied to different types of ultrasound equipment.

The above provides a detailed description of the preferred embodiments of the present application. However, it should be understood that the present application is not limited to the disclosed embodiments, and those skilled in the art can make various equivalent modifications or substitutions without departing from the spirit of the application. Such equivalent modifications or substitutions are included within the scope of the claims of this application.

**Claims**

1. A method for evaluating a contact state of an ultrasound probe on the basis of soft tissue morphology, wherein, the method comprises the following steps:

S11: collecting ultrasound information of morphological responses of an object under test for different contact states;
S12: on the basis of a feature of soft tissue and a boundary feature of the soft tissue in the ultrasound information, recognizing a boundary position of the soft tissue in the ultrasound information;

S13: on the basis of the boundary position and a preset area division condition, determining an area of interest and a sampling sub-area in the area of interest;

S14: extracting a morphological parameter of the soft tissue from the selected area of interest;

S15: according to the morphological parameter, calculating a predicted value that represents the contact state of an ultrasound probe by using at least one morphological response of the soft tissue;

S16: converting the predicted value into a time-varying indication signal.

2. The method according to claim 1, wherein, the ultrasonic information in S11 includes structural imaging information of the object under test, including ultrasound signals, one-dimensional ultrasound images (A-mode and M-mode images), two-dimensional ultrasound images (B-mode images, Doppler images, elastography images), and three-dimensional ultrasound images.

3. The method according to claim 1, wherein, the soft tissue in S12 refers to a type of biological tissue in the object under test that has physical contact with the ultrasound probe and is capable of undergoing varying degrees of morphological changes to different contact states based on its own physical characteristics, the soft tissue includes at least one of the following: skin, subcutaneous fat, fascia, and muscles; if the ultrasonic information collected in step S11 is in the form of ultrasound signals, based on the feature of at least one soft tissue and the boundary feature of the soft tissue corresponding to the ultrasound signal, using signal processing techniques to identify the boundaries of the soft tissue, respectively; if the ultrasonic information collected in step S11 is in the form of ultrasound images, based on the feature of at least one soft tissue and the boundary feature of the soft tissue corresponding to the ultrasound images, using image processing techniques.

4. The method according to claim 1, wherein, the area of interest in S13 includes at least one soft tissue, which is delineated by the boundary of a selected single soft tissue or the boundaries of a combination of multiple soft tissues; the soft tissue refers to a type of biological tissue in the object under test that has physical contact with the ultrasound probe and is capable of undergoing varying degrees of morphological changes to different contact states based on its own physical characteristics, and is **characterized by** being located in a relatively superficial region and the tissue structure, location, adjacent structures, and their hierarchical relationships of it being displayed by the spatial resolution of existing ultrasound imaging; the sampling sub-area is obtained by dividing the selected area of interest to get at least one sub-area for collecting morphological parameters at specific spatial positions.

5. The method according to claim 3 or 4, wherein, the morphological parameters of the soft tissues in S14 include at least one of the following: thickness, cross-sectional area, pennation angle, and the length of muscle fiber; the area of interest contains multiple types of soft tissues, the morphological parameter is the sum of the corresponding morphological parameter values for each soft tissue; the identification of boundaries of the soft tissues and extraction of morphological parameters are performed in real-time based on each frame of the image, synchronized with the image display.

6. The method according to claim 5, wherein, the predicted values in S15 are conversions of morphological parameters that characterize relative pressure and relative perpendicularity of the ultrasound probes using the deformation degree of at least one soft tissue; the predicted values are directly represented by the absolute value of the morphological parameters, or calculated by mathematical statistics method using at least one real-time morphological parameter, including change rate, ratio, difference, or other measurement indicators obtained through mathematical formulas or models;

translating the morphological parameters into predicted values using preset mathematical statistical methods, and the predicted values are relative values;

or directly representing the predicted values by the absolute value of the morphological parameters.

7. The method according to claim 6, wherein, the predicted values are calculated using a combination of other parameters according to certain weights or mathematical statistical methods, the parameters include at least one of the following: physiological parameters, image feature parameters, pressure parameters, and motion parameters;

when the parameter is a physiological parameter, the blood flow velocity, blood flow direction, and blood flow intensity of the object under test are extracted from the Doppler images;

when the parameter is an image feature parameter, color feature parameters, texture feature parameters, shape feature parameters, and spatial relationship feature parameters are extracted from the ultrasound images;

when the parameter is a pressure parameter, the pressure is collected from the pressure detection components;

when the parameter is a motion parameter, the angular velocity, acceleration, velocity, and angle are collected from spatial sensors.

8. The method according to claim 1, wherein, the indication signal in S16 refers to real-time data of the pressure and perpendicularity between the probe and the surface of the object under test, and is used for observation and can include at least one of the following: tactile indication, auditory indication, and visual indication, the carrier of the indication signal is an indicator using to output instantaneous indication signals.

9. The method according to claim 8, wherein, the indicator includes software indicators and hardware indicators;

software indicators include indicators displayed through color blocks, color bars, dials, scales, lights, counters, graphs, maps, or other charts and are set in any region of the virtual user interface and are displayed together with the ultrasound images;

hardware indicators are indicators that provide indication signals through numerical displays, lights, images, sounds, or vibrations, and the hardware indicators are physically positioned within the ultrasound hardware apparatus at a specific location.

10. The method according to claim 1, wherein, further comprises: comparing the predicted values with the preset conditions, and are generating corresponding instructions based on instantaneous comparison results;

wherein, obtaining the instantaneous comparison results by evaluating the relationship between the predicted values calculated from the current frame of ultrasound images and the preset reference data, determining whether the contact state of the probe meets the preset conditions, and generating corresponding instructions based on determination results; this step is synchronized with S15.

11. The method according to claim 10, wherein, after comparing the predicted values with the preset conditions, and are generating corresponding instructions based on instantaneous comparison results, the following steps are also included:

based on the predicted value, compensating for measured values obtained by the ultrasound equipment;

the measured values are obtained by existing ultrasound equipment, and the specific measurement function depends on the hardware and algorithm of the existing ultrasound equipment.

12. The method according to claim 10, wherein, the instructions include alarm notification instructions or trigger sampling instructions; the comparison calculations performed before generating the instructions are based on real-time analysis of each frame of ultrasound images;

the sampling involves collecting ultrasound images and performing functional measurements;

the expression forms of alarm notifications can include at least one of the following: tactile feedback, auditory cues, and visual cues.

13. The method according to one of claims 1-9, wherein, the method also includes using the morphological responses of the ultrasound gel pad to evaluate the contact state of the probe, specifically including:

S31: collecting the ultrasound information of the morphological response of the ultrasound gel pad and the object under test to different contact states;

S32: based on the features of the corresponding ultrasound gel pad and/or soft tissue as well as the boundary features of the ultrasound gel pad and/or soft tissue in the ultrasound images or signals, using signal or image processing techniques to determine the boundary positions of the ultrasound gel pad and/or soft tissue from each frame of the ultrasound image;

S33: based on the boundary of the ultrasound gel pad and/or soft tissue, as well as the preset area division condition, determining the area of interest and its sampling sub-area;

S34: extracting the morphological parameters of the ultrasound gel pad and/or soft tissue in each sampling sub-area from the selected area of interest;

S35: based on the morphological parameters and preset mathematical methods, calculating the predicted value using the morphological responses of the ultrasound gel pad and/or soft tissue to characterize the contact state of the probe;

S36: converting the predicted value into the time-varying indication signal;

wherein, the area of interest is delineated by the boundary of the ultrasound gel pad and/or soft tissue; the morphological parameters include the thickness and cross-sectional area of the ultrasound gel pad and/or soft tissue.

14. The method according to claim 6, wherein, the predicted values in S15 are conversions of morphological parameters that characterize relative pressure and relative perpendicularity of the ultrasound probes using the deformation degree of at least one soft tissuemorphological parameter, the calculation method is as follows,

selecting thickness as a morphological parameter and thickness change rate as the predicted value;
calculating the predicted value using the following change rate formula;

$$Z = \frac{Y_{deformation\ state} - Y_{unloaded\ state}}{Y_{unloaded\ state}} * 100\%$$

wherein, the predicted value z represents the degree of change in soft tissue thickness relative to the unloaded state;
Y represents the overall thickness of the area of interest, in the unloaded state, the soft tissues are in a state of static relaxation, with no probe contact or minimal contact with the probe; in the deformed state, the area of interest is under the pressure of the probe;
if the predicted values collected from three sampling sub-areas are Z1, Z2, and Z3, the relative pressure value F can be calculated using the following formula $F = \frac{Z_1 + Z_2 + Z_3}{3} * 100\%$ ; the relative pressure value F represents the percentage change in soft tissue morphology and indicates the relative pressure applied by the probe on the body surface; the relative pressure value F also represents the average pressure;

the relative value of the perpendicularity P can be calculated as follows $P = \frac{(|Z_1 - F| + |Z_2 - F| + |Z_3 - F|)}{3F}$
; the relative value of the perpendicularity P represents the difference in morphological response between different sampling sub-areas and can reflect the relative perpendicularity of the probe to the body surface; when P is larger, it indicates an increased difference in morphological response between the sampling sub-areas, and the tilt angle of the probe to the body surface deviates further from vertical.

15. The method according to claim 6 or 7, wherein, the predicted value of S15 can also be obtained by combining different parameters, which are at least one of the soft tissue morphological parameters related to ultrasound images, including

the parameters involved in the weighted calculation include morphological parameter x1, the total thickness of skin and subcutaneous fat,
the pressure parameter x2,
the image feature parameter x3 which is the ultrasound features of soft tissues,
the motion parameter x4, which is the probe attitude;
the predicted value is calculated by weighted average model:

suppose there are n parameters [x1, x2, x3, ..., xn] (n≥1) in the combination, and each parameter has a weight w, that is, [w1, w2, w3, ..., wn] (n≥1, wn is a constant), then the weighted average is

$$x = \frac{w1 * x1 + w2 * x2 + ... + wn * xn}{w1 + w2 + ... + wn} ;$$

if based on the given weight, the predicted value after weighted processing is

$$\star = \frac{50\% * x1 + 20\% * x2 + 20\% * x3 + 10\% * x4}{50\% + 20\% + 20\% + 10\%};$$

it should be understood that the type and quantity of parameters, as well as the weights, are not limited.

16. An apparatus for the method for evaluating a contact state of an ultrasound probe on the basis of soft tissue morphology according to claim 1, wherein, comprises:

an information acquisition unit for receiving an ultrasound information of an object under test;
a contact state measurement unit;
a contact state indication unit for converting a predicted value to a time-varying indication signal, and providing real-time contact state information between an ultrasound probe and a body surface through the indicator;
the contact state measurement unit includes:

a soft tissue boundary recognition unit for determining boundary positions of at least one type of soft tissue in the object under test based on the feature of at least one soft tissue and the boundary feature of the soft tissue corresponding to the ultrasound images or signals using signal or image processing techniques;
an area of interest determination unit for determining an area of interest and a sampling sub-area in the area of interest on the basis of the boundary position and a preset area division condition;
a soft tissue morphology quantification unit for calculating the morphological parameters of soft tissue in each sampling sub-area from the selected area of interest;
a relative pressure calculation unit for converting morphological parameters into predicted values using at least one soft tissue morphology response to characterize the relative pressure of the probe according to certain mathematical statistical methods;
a relative perpendicularity calculation unit for converting morphological parameters into predicted values that use at least one soft tissue morphology response to characterize the relative perpendicularity of the probe according to certain mathematical statistical methods.

17. The apparatus according to claim 16, wherein, the contact state measurement unit includes an added pressure compensation unit and a perpendicularity compensation unit which are respectively used to compensate for the measurement values obtained by the ultrasound equipment based on the relative pressure information and relative perpendicularity information calculated by the relative pressure calculation unit and the relative perpendicularity calculation unit.

18. The apparatus according to claim 16 or 17, wherein, further comprises:

a reminder unit for determining whether the real-time probe contact state meets the preset conditions and generating corresponding alarm prompts;
a trigger unit for issuing trigger instructions to existing ultrasound equipment, and instructing it to perform sampling on the object under test.

collecting ultrasound information of morphological responses of an object under test for different contact states — S11

on the basis of a feature of soft tissue and a boundary feature of the soft tissue in the ultrasound information, recognizing a boundary position of the soft tissue in the ultrasound information — S12

on the basis of the boundary position and a preset area division condition, determining an area of interest and a sampling sub-area in the area of interest — S13

extracting a morphological parameter of the soft tissue from the selected area of interest — S14

according to the morphological parameter, calculating a predicted value that represents the contact state of an ultrasound probe by using at least one morphological response of the soft tissue — S15

converting the predicted value into a time-varying indication signal — S16

Figure 1

collecting ultrasound information of morphological responses of an object under test for different contact states — S21

on the basis of a feature of soft tissue and a boundary feature of the soft tissue in the ultrasound information, recognizing a boundary position of the soft tissue in the ultrasound information — S22

on the basis of the boundary position and a preset area division condition, determining an area of interest and a sampling sub-area in the area of interest — S23

extracting a morphological parameter of the soft tissue from the selected area of interest — S24

according to the morphological parameter, calculating a predicted value that represents the contact state of an ultrasound probe by using at least one morphological response of the soft tissue — S25

converting the predicted value into a time-varying indication signal — S26

comparing the predicted values with the preset conditions, and are generating corresponding instructions based on instantaneous comparison results — S27

Figure 2

collecting the ultrasound information of the morphological response of the ultrasound gel pad and the object under test to different contact states — S31

based on the features of the corresponding ultrasound gel pad and/or soft tissue as well as the boundary features of the ultrasound gel pad and/or soft tissue in the ultrasound images or signals, using signal or image processing techniques to determine the boundary positions of the ultrasound gel pad and/or soft tissue from each frame of the ultrasound image — S32

based on the boundary of the ultrasound gel pad and/or soft tissue, as well as the preset area division condition , determining the area of interest and its sampling sub-area — S33

extracting the morphological parameters of the ultrasound gel pad and/or soft tissue in each sampling sub-area from the selected area of interest — S34

based on the morphological parameters and preset mathematical methods, calculating the predicted value using the morphological responses of the ultrasound gel pad and/or soft tissue to characterize the contact state of the probe — S35

converting the predicted value into the time-varying indication signal — S36

Figure 3

| | |
|---|---|
| collecting ultrasound information of morphological responses of an object under test for different contact states | S41 |

| | |
|---|---|
| on the basis of a feature of soft tissue and a boundary feature of the soft tissue in the ultrasound information, recognizing a boundary position of the soft tissue in the ultrasound information | S42 |

| | |
|---|---|
| on the basis of the boundary position and a preset area division condition, determining an area of interest and a sampling sub-area in the area of interest | S43 |

| | |
|---|---|
| extracting a morphological parameter of the soft tissue from the selected area of interest | S44 |

| | |
|---|---|
| according to the morphological parameter, calculating a predicted value that represents the contact state of an ultrasound probe by using at least one morphological response of the soft tissue | S45 |

| | |
|---|---|
| converting the predicted value into a time-varying indication signal | S46 |

| | |
|---|---|
| comparing the predicted values with the preset conditions, and are generating corresponding instructions based on instantaneous comparison results | S47 |

| | |
|---|---|
| based on the predicted value, compensating the measured values, ultrasound signals or ultrasound images obtained by the ultrasound equipment | S48 |

Figure 4

Figure 5

```
                          ┌─────────────────────┐
                          │                     │
                          │   place the probe   │
                          │                     │
                          └─────────────────────┘
```

| morphological parameter $X_1$ | morphological parameter $X_2$ | morphological parameter $X_3$ | morphological parameter $X_4$ |
| --- | --- | --- | --- |
| weight $W_1$ | weight $W_2$ | weight $W_3$ | weight $W_4$ |

```
                     ┌─────────────────────┐
                     │ Predicted value X   │
                     │  after combination  │
                     └─────────────────────┘
```

Figure 6

(t₁)                    (t₂)

Figure 7

502

contact state
measurement unit

504

soft tissue
boundary
recognition unit

505

area of interest
determination unit

soft tissue
morphology
quantification unit

506

501

information
acquisition unit

relative pressure
calculation unit

relative
perpendicularity
calculation unit

503

contact state
indication unit

507

508

Figure 8

Figure 9

Figure 10

Figure 11

## INTERNATIONAL SEARCH REPORT

| International application No. |
|---|
| **PCT/CN2021/103789** |

| A. | CLASSIFICATION OF SUBJECT MATTER |
|---|---|
| | A61B 8/00(2006.01)i;  A61B 8/06(2006.01)i |

According to International Patent Classification (IPC) or to both national classification and IPC

| B. | FIELDS SEARCHED |
|---|---|

Minimum documentation searched (classification system followed by classification symbols)

    A61B

Documentation searched other than minimum documentation to the extent that such documents are included in the fields searched

Electronic data base consulted during the international search (name of data base and, where practicable, search terms used)

    CNTXT, CNKI, WPI, EPODOC: 意领科技有限公司, 黄子豪, 王立科, 郑永平, 郑乐勤, 超声, 探头, 软组织, 形态, 边界, 形态学, 时间, 信号, 图像, 图象, 感兴趣区域, 压力, 生理, 血流, 速度, 强度, 接触状态, 探头状态, ultrasound, probe, soft, tissue, form, boundary, imag+, pressure, speed, contact, state, judg+

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 103402436 A (FUJI PHOTO FILM CO., LTD.) 20 November 2013 (2013-11-20) description paragraph [0083], figures 1-3 | 1-18 |
| Y | CN 112089442 A (EIELING TECHNOLOGY LIMITED) 18 December 2020 (2020-12-18) description, paragraphs [0049]-[0149], and figures 1-12 | 1-18 |
| A | CN 109788944 A (FUJI PHOTO FILM CO., LTD.) 21 May 2019 (2019-05-21) entire document | 1-18 |
| A | CN 105030275 A (JIANG, Dianwei) 11 November 2015 (2015-11-11) entire document | 1-18 |
| A | US 2020077976 A1 (HITACHI, LTD.) 12 March 2020 (2020-03-12) entire document | 1-18 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 October 2021** | **26 October 2021** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<div align="center">

**INTERNATIONAL SEARCH REPORT**
**Information on patent family members**

</div>

International application No.

**PCT/CN2021/103789**

| Patent document cited in search report | | | Publication date (day/month/year) | Patent family member(s) | | | Publication date (day/month/year) |
|---|---|---|---|---|---|---|---|
| CN | 103402436 | A | 20 November 2013 | US | 2013338478 | A1 | 19 December 2013 |
| | | | | WO | 2012114729 | A1 | 30 August 2012 |
| | | | | JP | 2012187389 | A | 04 October 2012 |
| | | | | EP | 2679161 | A1 | 01 January 2014 |
| CN | 112089442 | A | 18 December 2020 | None | | | |
| CN | 109788944 | A | 21 May 2019 | JP | 6637611 | B2 | 29 January 2020 |
| | | | | WO | 2018055819 | A1 | 29 March 2018 |
| | | | | US | 2019192121 | A1 | 27 June 2019 |
| | | | | EP | 3517046 | A1 | 31 July 2019 |
| CN | 105030275 | A | 11 November 2015 | None | | | |
| US | 2020077976 | A1 | 12 March 2020 | None | | | |

Form PCT/ISA/210 (patent family annex) (January 2015)